Europäisches Patentamt

European Patent Office

Office européen des brevets

(11) Publication number: **0 225 177 B1**

# EUROPEAN PATENT SPECIFICATION

(45) Date of publication of patent specification: **01.09.93** (51) Int. Cl.5: **C12N 15/12**, C12N 9/64, C12N 1/20, C12N 5/00, C12P 21/02, A61K 37/54

(21) Application number: **86309250.8**

(22) Date of filing: **27.11.86**

The file contains technical information submitted after the application was filed and not included in this specification

(54) **DNA sequence coding for human tissue plasminogen activator.**

(30) Priority: **27.11.85 JP 264918/85**

(43) Date of publication of application:
**10.06.87 Bulletin 87/24**

(45) Publication of the grant of the patent:
**01.09.93 Bulletin 93/35**

(84) Designated Contracting States:
**BE CH FR GB IT LI NL SE**

(56) References cited:
EP-A- 0 093 619          EP-A- 0 178 105
EP-A- 0 211 260          EP-A- 0 213 794
WO-A-86/05514          DE-A- 3 537 176

**PROCEEDINGS OF THE NATIONAL ACADEMY OF SCIENCES OF THE USA, vol. 81, no. 17, September 1984, pages 5355-5359, Washington, US; T. NY et al.: "The structure of the human tissue-type plasminogen activator gene: correlation of intron and exon structures to functional and structural domains"**

(73) Proprietor: **MITSUI TOATSU CHEMICALS, Inc.
2-5 Kasumigaseki 3-chome
Chiyoda-Ku Tokyo 100(JP)**

(72) Inventor: **Takahashi, Seishi
1071-2-302, Nakanocho Totsuka-ku
Yokohama-shi Kanagawa-ken(JP)**
Inventor: **Morimoto, Kazushi
4-1409-933, Shiomidai 1-chome Isogo-ku
Yokohama-shi Kanagawa-ken(JP)**
Inventor: **Kusuhara, Nobumi
4-17, Mitsui Toatsu Iijima Apaato 2882,
Iijimacho
Totsuka-ku Yokohama-shi Kanagawa-ken(JP)**
Inventor: **Tomioka, Noboru
9, Nakanodaishataku 226-1, Takashi
Mobara-shi Chiba-ken(JP)**
Inventor: **Makino, Tadashi
19-11, Higiriyama 3-chome Kohnan-ku
Yokohama-shi Kanagawa-ken(JP)**
Inventor: **Omae, Fumio 4-1-7, Mitsui Toatsu
Kosugaya Apaato
2070, Iijimacho Totsuka-ku
Yokohama-shi Kanagawa-ken(JP)**

GENE, vol. 33, no. 3, April 1985, pages 279-284, Elsevier Science Publishers, Amsterdam, NL; M.J. BROWNE et al.: "Isolation of a human tissue-type plasminogen-activator genomic DNA clone and its expression in mouse L cells"

FEBS LETTERS, vol. 189, no. 1, September 1985, pages 145-149, Elsevier Science Publishers B.V., Amsterdam, NL; H. KAGITANI et al.: "Expression in E. coli of finger-domain lacking tissue-type plasminogen activator with high fibrin affinity"

DNA, vol. 4, no. 6, 1985, pages 419-428, Mary Ann Libert, Inc., Publishers; J.F. LEMONTT et al.: "Expression of active human uterine tissue plasminogen activator in yeast"

CHEMICAL ABSTRACTS, vol. 100, no. 23, 4th June 1984, page 252, abstract no. 187922a, Columbus, Ohio, US; G. POHL et al.: "Differences between uterine and melanoma forms of tissue plasminogen activator"

(74) Representative: Holdcroft, James Gerald, Dr. et al
Graham Watt & Co., Riverhead
Sevenoaks, Kent TN13 2BN (GB)

## Description

This invention relates to a novel DNA sequence containing the DNA sequence coding for human tissue plasminogen activator produced by human normal cells, a recombinant DNA including the DNA sequence, host cells transformed with the recombinant DNA, and a process for producing human tissue plasminogen activator by use of the host cells.

In the treatment of thrombosis, urokinase and streptokinase are currently used for reasons of convenience. However, they involve the problems of hemorrhage and antigenicity, and have a weak affinity for fibrin.

Moreover, urokinase is being produced chiefly by extracting crude urokinase from human urine and then purifying it. However, this production process has the disadvantages that it is difficult to secure a constant supply of human urine and that the process steps are not hygienic.

Consequently, various attempts have been made to develop substitute drugs. In the present situation, however, no thrombolytic agent that can solve the above-described problems and is satisfactory from the viewpoints of safety and mass production is available for practical purposes.

As a substitute for conventional thrombolytic agents such as urokinase and the like, attention is being paid to tissue plasminogen activator (hereinafter referred to as t-PA) that plays a major role in the in vivo fibrinolysis reaction and has a high affinity for fibrin. EP-A-0093619, EP-A-0178105, DE-A-3537176 disclose human tPAs, but none disclose the particular t-PA of the invention having a distinct amino acid sequence, or suggest the use of the particular expression system of the invention. FEBS Lett. 168, 29-32 (1984) discloses a human tPA having the same N-terminal amino acid sequence as the tPA of the invention.

There are a number of well-known methods for the production of t-PA. They include a cell culture process using Bowes melanoma derived from malignant melanosarcoma cells which is a kind of cancer, and a process comprising the steps of isolating a DNA fragment containing the DNA sequence coding for t-PA from Bowes melanoma, incorporating this DNA fragment into an expression vector to form a recombinant DNA, transforming host cells with this recombinant DNA, and culturing the transformed host cells to cause them to produce t-PA.

However, the mechanism of carcinogenesis has not yet been adequately elucidated from a scientific point of view and, therefore, no definite answer has been given about the safety of the steps in the above-described processes using cancer cells and about the safety of the resulting t-PA for use as a drug. For these reasons, a careful and thorough examination is still required in practicing the above-described processes and in using the t-PA produced thereby.

In view of the above-described problems of the prior art, the present inventors made an extensive study with special attention to human normal cells which can eliminate the risk associated with the use of cancer-derived cell lines such as Bowes melanoma, and in search of human normal cells which can be used to obtain the DNA coding for t-PA.

As a result, the present inventors have found that a cell line consisting of human fetal epithelial cells highly productive of t-PA can be derived from human fetal epithelial cells and that, according to a recombinant DNA technique using this cell line, a novel DNA sequence containing the DNA sequence coding for t-PA can be constructed, this DNA sequence can be incorporated into a replicable vector which permits the expression of t-PA, and the recombinant DNA so formed can be used to obtain transformed host cells useful in the production of t-PA.

Accordingly, it is an object of the present invention to provide the DNA sequence coding for t-PA which can solve the above-described problems of conventional drugs such as urokinase and the like, can eliminate the risk associated with the use of cells (e.g., Bowes melanoma) derived from cancer cells, and can suitably be used in the mass production of t-PA; a recominbant DNA incorporating the DNA sequence; and host cells transformed with the recombinant DNA.

The DNA sequence coding for human t-PA which can be isolated in the manner described in detail hereinbelow.

The invention also comprehends a process for the production of human tissue plasminogen activator comprising the step of culturing the mammalian cell of the invention in the presence of a metal ion as an inducer for the mouse metallothionein promoter or the human metallothionein promoter.

There now follows a description of embodiments of the invention. This description is given by way of example of the invention only and not by way of limitation thereof.

In the accompanying drawings:-

Fig. 1 illustrates a DNA base sequence containing the DNA base sequence coding for human tissue plasminogen activitator produced by human fetal epithelial cells in accordance with the present invention and indicates that this DNA base sequence contains a 5' untranslated region, a signal sequence, the DNA sequence coding for t-PA, and a 3' untranslated region;

Fig. 2 is a diagram illustrating the procedure for preparing the plasmid pMT-1 described in Example 2;

Fig. 3 is a diagram illustrating the procedure for preparing the plasmid phT-1 described in Example 3.

According to the present invention, a DNA sequence containing the DNA sequence coding for human t-PA produced by human fetal epithelial cells can be obtained by use of any of various cell strains derived from human fetal epithelial cells, and having the ability to produce t-PA.

Such cell strains can be obtained by growing various types of human fetal epithelial cells (preferably, human fetal epidermal cells, fetal pulmonary cells, fetal renal cells and the like) and then screening them for the ability to produce t-PA. Among others, there may preferably be used cell strains which have greater ability to produce t-PA and consist of human normal diploid cells having the diploid number (2n = 46) of chromosomes and having undergone a finite number of subcultures.

One exemplary screening technique is the method of A. Granelli-Piperno and E. Reich [J. Exp.Med., 148, 223 (1978)] as used in the examples which will be given later. This method is characterized in that fibrinolytic activity on a fibrin plate is utilized as an index for the ability to produce t-PA.

In order to obtain the DNA sequence of the present invention from a cell strain having the ability to produce t-PA, there may be used any of various genetic engineering techniques. However, the procedure which has been established by the present inventors and will be specifically described hereinbelow is especially useful.

Purification and size fractionation of mRNA

Total RNA is first extracted from cells having the ability to produce human t-PA. Then, oligo(dT)-cellulose is used to separate and recover poly(A) plus mRNA from the total RNA. The resulting mRNA is subjected to agarose gel electrophoresis and thereby fractionated according to the size. The resulting fractions are separately microinjected into oocytes of Xenopus laevis, which are separately cultivated in Barth's medium [J.B. Gurdon, The Control of Gene Expression in Animal Development, Oxford University Press, 1974].

After completion of the cultivation, the fraction containing the desired mRNA having a portion corresponding to the DNA sequence coding for human t-PA is selected by assaying the human t-PA activity of each culture medium according to the aforesaid method using a fibrin plate. The presence of the desired mRNA in this fraction can be confirmed, for example, by the fact that, if both a sample of the culture medium of the oocyte having that fraction introduced thereinto and an anti-human t-PA serum are placed on a fibrin plate, the fibrinolysis reaction on the fibrin plate is hindered by the anti-human t-PA serum.

Then, the desired DNA sequence is obtained by using the selected fraction containing the mRNA having a portion corresponding to the desired DNA sequence coding for t-PA. To this end, the aforesaid fraction is used to synthesize cDNAs corresponding to the mRNAs present in the fraction and the cDNA containing the desired DNA sequence is selected from among those cDNAs with the aid of a DNA probe. This procedure will be more fully described hereinbelow.

Construction of a cDNA library containing the t-PA gene

The mRNA fraction exhibiting human t-PA activity and selected in the above-described manner is used to synthesize cDNAs corresponding to the mRNAs present in the fraction. This can be accomplished, for example, by the method of Gubler and Hoffman [U, Gubler and B.J. Hoffman, Gene, 25, 263 (1983)]. The cDNAs thus obtained are inserted into the plasmid pUC13.

Then, the mixture of the plasmid pUC13 having various cDNAs inserted thereinto is introduced into E. coli HB101 (ATCC 33694). Thus, ampicillin-resistant colonies are obtained.

As the plasmid (vector) into which cDNAs are inserted and the host bacterium into which the plasmid is introduced, any other plasmid (vector) and host bacterium may be chosen according to the need, provided that they can provide a desired cDNA clone containing the DNA sequence coding for human t-PA.

From each of the colonies thus obtained, a part of the cells are transferred to a Whatman 541 filter. Then, according to the method of Gergen et al. [J.P. Gergen et al., Nucleic Acids Res., 7, 2115 (1979)), the DNA derived from each colony is immobilized on the filter to obtain a cDNA library.

Preparation of a DNA probe

First, the previously selected human normal cells productive of t-PA are grown in culture. Then, according to the method described in European Patent Application No. 83903315.6 (WO 84/01714), the human t-PA so produced is recovered from the culture medium and purified for use in the preparation of a DNA probe.

Then, in order to prepare a probe, partial amino acid sequences of this human t-PA are determined by partially hydrolyzing the human t-PA protein with trypsin, separating and purifying the hydrolyzates, and analyzing their amino acid sequences from the amino end. Subsequently, the optimum region for the preparation of a probe is selected from among the amino acid sequences determined in the above-described manner. As the optimum region, the present inventors have selected the sequence portion of Tyr-Cys-Trp-Cys-Asn.

Next, 8 types of 14-nucleotide oligomers (l4mers) which are complementary to the DNA sequence coding for the above amino acid sequence, i.e.

$$5'-dTT \binom{A}{G} CACCA \binom{A}{G} CA \binom{A}{G} TA,$$

are synthesized according to the solid-phase phosphotrieter method [Crea et al., Nucleic Acids Res., $\underline{8}$, 2331 (1980)].

Isolation and identification of bacterial clones containing the t-PA cDNA sequence

A mixture of the eight types of l4-nucleotide oligomers (l4mers) obtained in the above-described manner is treated with T4-polynucleotide kinase to label their 5'-end with $^{32}$P. This mixture, which serves as a probe, is hybridized to the previously prepared cDNA library.

Then, according to the method of Birnboim and Doly [Nucleic Acid Res., 7, 1513 (1979)], plasmid DNA is isolated from the colonies constituting the sources of DNA exhibiting a positive hybridization reaction. The base sequence of its cDNA insert is determined by the method of Maxam and Gilbert [Proc. Natl. Acad. Sci. U.S.A., 74, 560(1977)]. The presence of the desired DNA sequence in the cDNA insert of this isolated plasmid DNA can be confirmed by comparing the amino acid sequence derived from the above-determined base sequence of the cDNA insert with the previously determined amino acid sequence of purified human t-PA.

The cDNA isolated and identified in the above-described manner contains the DNA sequence coding for human t-PA and have been constructed by the present inventors for the first time. The provision of such a novel DNA sequence by the present inventors has made it possible to produce human t-PA by use of host cells transformed with a recombinant DNA containing the DNA sequence coding for human t-PA which can solve the above-described various problems of the prior art. This process for producing human t-PA will be more fully described hereinbelow.

On the basis of the following findings obtained by the present inventors, the host cells used in the present process for producing human t-PA comprise mammalian cells.

Specifically, the whole amino acid sequence of human t-PA originating from human fetal epithelial cells was derived from the previously determined DNA sequence of human t-PA. By examining this amino acid sequence, it has been presumed that the human t-PA molecule has 4 sites to which sugar chains can be added. In fact, the molecular weight of human t-PA is reduced to about 50000 when a cell strain productive of human t-PA is grown in the presence of tunicamycin which is an antibiotic substance inhibiting the addition of sugar chains. Accordingly, sugar is thought to comprise about 30% of the molecular weight. Considering that the addition of sugar chains may participate in the stable in vivo functioning of the enzyme and the enzyme having no sugar chains may exhibit antigenicity in vivo, the present inventors have concluded that it would be better to cause the cloned human t-PA gene to be expressed in mammalian cells and thereby produce the natural form of human t-PA having sugar chains added thereto. For this reason, mammalian cells are used as hosts.

The mammalian cells used as hosts may be selected from a variety of presently available established cell lines of mammalian origin.

Preferred examples thereof include mouse L cells; thymidine kinase deficient L cells; dihydrofolate reductase deficient, Chinese hamster ovary (CHO) cells; mouse Cl27I cells (ATCC CRLl6l6); mouse NIH3T3 cells (ATCC CRLl658); mouse FM3A cells; human Hela cells (ATCC CCL2); African green monkey COSI cells (ATCC CCLl650); various types of mouse and human myeloma cells such as mouse P3/NSl/I-Ag4-I (NS-I) cells (ATCC TIBl8), mouse P3X63Ag8 cells (ATCC TIB9) and mouse J558 cells (ATCC TIB6); and the like. It is to be understood that the foregoing cells are given only for illustrative purposes and other types of mammalian cells may also be used.

As the expression vector into which the DNA sequence coding for human t-PA is incorporated, there may be used any vector that can replicate within the host cells used and can transform the host cells so as to permit the expression of the gene coding for human t-PA. Among others, there may preferably be used vectors containing a promoter region, a ribosome binding site, a termination sequence, a polyadenylated site, a replication origin, and a gene which serves as a selectable marker when host cells are transfected with the vector.

As the ribosome binding site present in the aforesaid expression vectors, there may generally be used those derived from viruses of mammalian origin. For example, the promoter regions and ribosome binding sites possessed by papovaviruses, adenoviruses, retroviruses, human cytomegaloviruses and the like are usable. Moreover, the ribosome binding site present in an upstream portion of genes derived from mammalian cells can also be used. The promoter regions and ribosome binding sites of the mouse and human genes for metallothionein are used.

As the termination sequence and polyadenylated site, there may likewise be used those derived from viruses and various genes.

As to the replication origin, the vector may be constructed so as to contain an exogenous origin which can be derived from a virus as described above, particularly bovine papilloma virus (hereinafter referred to as BPV) or simian virus 40 (hereinafter referred to as SV 40). Alternatively, the desired expression vector may be incorporated as a fragment into a chromosome of the host cell so as to utilize the replication mechanism of the chromosome.

The gene which serves as a selectable marker when host cells are transfected with the vector is not always required. However, its presence is advantageous in that the transformed cells can be selected rapidly and accurately.

The genes useful for this purpose include, for example, the thymidine kinase gene (TK) of herpes simplex virus (HSV) and the dihydrofolate reductase gene (DHFR) of mice. In these cases, a thymidine kinase deficient (tk⁻) strain or a dihydrofolate reductase deficient (dhfr⁻) strain should be used as host cells.

In addition, there may also be used the xanthine guanine phosphoribosyltransferase gene (Eco-gpt) of Escherichia coli exhibiting resistance to mycophenolic acid in mammalian cells, and the neomycin resistance gene (nec) derived from transposon 5 exhibiting resistance to the antibiotic substance G4l8 in mammalian cells.

The expression vector, which preferably contains the above-described various regions, can be constructed according to well-known genetic recombination techniques.

In order to transfect host cells with the vector of the present invention containing the DNA sequence coding for human t-PA, there may be used any of various methods such as treatment with calcium phosphate [F.L. Graham and A.J. Van der Eb, Virology, 52, 546 (1978)], injection into the nucleus [A. Graessman et al., Methods in Enzymology, 65, 816 (1980), Academic Press, New York], protoplast fusion [W. Schaffner, Proc. Nat]. Acad. Sci. USA, 77, 2163 (1980)], introduction by an electric shock [H. Potter et al., Proc. Natl. Acad. Sci USA, 81, 7161 (1984)], treatment with DEAE-dextran [P. Sheldrick et al., Proc. Natl. Acad. Sci. USA, 70, 3621 (1973)] and the like.

After the transfection is carried out according to any of the above-described methods, the resulting transformed cells are separately cultured. Their production of human t-PA originating from human normal cells can be confirmed by assaying the human t-PA activity of the supernatant of such cultures.

Thus, human t-PA originating from human fetal epithelial cells can be mass-produced by culturing the transformed cells and thereby causing them to secrete human t-PA into the culture medium. Some typical embodiments of this process for producing human t-PA are more specifically described in the following examples.

Example 1 [Construction of a DNA sequence containing the DNA sequence coding for human t-PA]

1. Screening of human normal cells productive of human t-PA

The various types of human normal cells listed in Table 1 were subjected to a screening procedure. The cell lines established by the present inventors were obtained as follows: A piece of source tissue was minced with scissors or the like and then digested with trypsin, collagenase, dispase and the like to obtain dispersed and liberated cells. These cells were suspended in a culture medium and plated. Then, clones exhibiting active growth were isolated and repeatedly subcultured. Finally, strains which could be stably subcultured were selected and used as normal cell lines for screening purposes. The cell lines to be screened were grown until the confluent state was attained. Using 15 μl of the supernatant of each culture, the human t-PA activity of the supernatant was assayed. In this assay, 15 μl of the supernatant was placed on a fibrin plate prepared by the aforesaid method of Granelli-Piperno and Reich. After this fibrin plate was allowed to stand at 37°C for 16 hours, the resulting dissolution zone was measured. At the same time, a sample of the supernatant mixed with an anti-urokinase serum and a sample of the supernatant mixed with an anti-human t-PA serum were also prepared. The dissolution zones produced by these samples were also measured and compared with the dissolution zone produced by the sample containing no antiserum.

A standard curve was constructed by using purified urokinase as a standard and measuring the dissolution zones produced by its diluted solutions. Using this standard curve, fibrinolytic activity was expressed in terms of international urokinase units. Some of the screened cells and their human t-PA activities and urokinase activities are shown in Table 1. Thus, cell line MTC0l7 was found to produce and secrete human t-PA in relatively large amounts, so that this cell line was selected as the source material for the isolation of the human t-PA gene. This cell strain was derived from human fetal epithelium and consisted of normal diploid cells having the diploid number (2n = 46) of chromosomes and having undergone about 80 subcultures.

## Table 1

## Screening of Human Normal Cells Productive

## of Human t-PA

| Cell Line | Human t-PA activity (units/ml) | u-PA activity (units/ml) | Origin |
|---|---|---|---|
| MTC001 | 3 | 3 | Established by the present inventors. Human fetal lung. |
| MTC005 | 10 | 10 | " |
| MTC006 | 4 | 9 | " |
| MTC011 | 7 | 4 | Established by the present inventors. Human fetal prepuce. |
| MTC012 | 3 | 0 | " |
| MTC014 | 10 | 10 | Established by the present inventors. Human fetal epithelium. |
| MTC017 | 20 | 10 | " |
| MTC023 | 6 | 2 | Established by the present inventors. Human fetal kidney. |
| MTC025 | 0 | 12 | " |
| MTC029 | 5 | 5 | " |

- to be cont'd -

8

Table 1 (Cont'd)

| HEL299 | 3 | 2 | ATCC CCL137. Human fetal lung. |
|---|---|---|---|
| WI-38 | 0 | 0 | ATCC CCL75. Human fetal lung. |
| WISH | 0 | 0 | ATCC CCL25. Human amnion. |
| FL | 0 | 0 | ATCC CCL62. Human amnion. |
| Chang Liver | 0 | 0 | ATCC CCL13. Human liver. |

2. Purification and size fractionation of mRNA

Using Eagle's minimal essential medium containing 10% FCS, cell line MTC0l7 was cultured in large amounts to collect $5 \times 10^8$ cells. The collected cells were washed with 200 ml of phosphate-buffered saline (PBS) and then resuspended in 20 ml of a 10mM Tris-HCl buffer solution (pH 8.6) containing 10mM KCl, 1.5mM $MgCl_2$ and 3mM dithiothreitol. After the cells were lyzed by adding Nonidet P-40 to the suspension (so as to give a final concentration of 0.5%), the resulting solution was centrifuged and the supernatant containing total RNA was further purified by extraction with phenol-chloroform.

Then, sodium acetate was added to the aqueous phase so as to give a concentration of 0.3M, and the total RNA was precipitated by adding 2 volumes of ethanol to the solution. According to the method of T. Maniatis et al. [T. Maniatis et al., Molecular Cloning, 197-198, Cold Spring Harbor Laboratory, New York (1982)], the precipitate so formed was treated with oligo(dT)cellulose to separate mRNA from the total RNA. In the above-described manner, 4-5 mg of total RNA and 100-150 $\mu$g of poly(A) plus mRNA were obtained from $5 \times 10^8$ cultured cells.

According to the method of T. Maniatis et al. [T. Maniatis et al., Molecular Cloning, 204-205, Cold Spring Harbor Laboratory, New York (1982)], the poly(A) plus mRNA obtained in the above-described manner was fractionated in low-melting agarose gel. This fractionation was carried out by treating the poly-(A) plus mRNA with methylmercury hydroxide and then subjecting it to electrophoresis in a flat plate of gel containing 1.5% low-melting agarose, 50mM boric acid, 5mM sodium borate and 10mM sodium sulfate. After completion of the electrophoresis, the gel was immersed in a 0.1M dithiothreitol solution for 30 minutes and then sliced according to the magnitude of the molecular weight. Four volumes of 0.5M ammonium acetate was added to each slice and heated to 65°C until the gel was dissolved. Thereafter, the resulting solution was repeatedly extracted with phenol and with chloroform to remove the agarose components. Finally, the mRNA fraction was recovered by precipitation with ethanol.

Then, according to the above-described method of J.B. Gurdon, 50 ng of each mRNA fraction was microinjected into an oocyte of Xenopus laevis. The oocytes having mRNA introduced thereinto were placed in Barth's medium and allowed to stand at 20°C for 24 hours. Subsequently, a sample of each medium was placed on a fibrin plate prepared by the aforesaid method of Granelli-Piperno and Reich. This fibrin plate was incubated at 37°C for 16 hours and the resulting dissolution zone was measured. At the same time, a sample of the medium mixed with an anti-human t-PA serum was also incubated. Thus, the mRNA fraction which gave the largest dissolution zone and underwent a suppression of fibrinolytic activity in the presence of the anti-human t-PA serum was selected.

3. Construction of a cDNA library containing the t-PA gene.

According to the aforesaid method of Gubler and Hoffman, 5 μg of the fraction selected in the above-described manner and containing the mRNA coding for t-PA was used to prepare double-stranded cDNAs. After deoxy(C) residues were joined to each cDNA, the resulting cDNAs were annealed with 1 μg of the plasmid pUC13 having deoxy(G) residues joined thereto at the PstI site. Using the annealed mixture, E. coli HB101 (ATCC 33694) was transformed to obtain 5000 ampicillin-resistant colonies.

According to the method of Gergen et al., a portion of each colony was transferred to a Whatman 54I filter. Thereafter, the DNA of each colony was immobilized to the filter by subjecting it to a series of treatments including alkali treatment, neutralization, washing and drying.

4. Preparation of a DNA probe

Human t-PA was isolated from human normal cell strain MTC0I7 according to the method described in European Patent Application No. 83903315.6 (WO 84/01714) and comprising a combination of ion exchange chromatography, affinity chromatography, gel filtration and the like. For the purpose of preparing a DNA probe, partial amino acid sequences of human t-PA were determined in the following manner.

1 mg of purified human t-PA was dissolved in 3 ml of 0.1M ammonium bicarbonate (pH 7.5). To the resulting solution was added trypsin [treated with L-I-tosylamido-2-phenylethyl chloromethyl ketone (TPCK)] in a molar ratio of 100000:1. Then, partial hydrolysis was effected at room temperature for 2 hours. The reaction was stopped by adding diisopropyl fluorophosphate (DFP) thereto in an amount equal to 10000 times the number of moles of trypsin added. The reaction product was dialyzed against 5mM formic acid and then freeze-dried. Then, the freeze-dried protein was dissolved in 5 ml of a solution containing 0.56M Tris-HCl (pH 8.6), 8M urea and 5mM EDTA. To the resulting solution was added 0.1 ml of $\beta$-mercaptoethanol to reduce the disulfide bonds. The reaction was carried out at 45°C for 2 hours under an atmosphere of nitrogen. Then, the reduced disulfide bonds were carboxymethylated by adding 1.0 ml of a solution containing 1.4M iodoacetic acid and IN NaOH. After standing at room temperature for 20 minutes, the reaction mixture was dialyzed at room temperature against water containing 0.01% Tween 80 and then freeze-dried.

The freeze-dried carboxymethylated protein was dissolved in 5 ml of a 0.1M ammonium bicarbonate buffer solution (pH 7.2) containing 0.1M ammonium thiocyanate. The resulting solution was passed through a Red-Sepharose column which had been equilibrated with the same buffer solution. After the column was washed with the same buffer solution, the adsorbed protein was eluted by increasing the concentration of ammonium thiocyanate linearly to 1.0M. As a result, protein peaks were obtained in the unadsorbed fraction and the adsorbed fraction. When analyzed by SDS-acrylamide gel electrophoresis, each of the protein peaks gave a single protein band having a molecular weight of about 35,000.

Each of the protein peaks was collected, dialyzed against 0.1M ammonium bicarbonate (pH 7.5) and then freeze-dried.

The amino acid sequences of the purified human t-PA obtained from a culture of cell strain MTC 0I7 and the proteins obtained therefrom in the above-described manner were determined by means of a Gas-Phase Protein Sequencer (Applied Biosystem Co.).

For the purified human t-PA, the sequence of N-terminal 38 amino acid residues was determined and was found to include Gly-Ala-Arg-Ser-Tyr-Gln- in the N-terminal region. The amino acid sequence of the unadsorbed protein fraction started with Ile-Lys-Gly-Gly-leu-, and the adsorbed protein fraction had the amino acid sequence of

## Ser-Asn-Arg-Val-Glu-Tyr-Cys-

## Trp-Cys-Asn-Ser-Gly-Arg-.

The N-terminal Ser of this adsorbed protein fraction was found to be located at position 31 counting from the N-terminus of the purified human t-PA.

As a result, the amino acid sequence of Tyr-Cys-Trp-Cys-Asn was selected as the optimum region for the preparation of a probe.

Thus, 8 types of I4-nucleotide oligomers (I4mers) which were complementary to the DNA sequence coding for the above amino acid sequence, i.e.

10

$$\text{dTT} \binom{A}{G} \text{CACCA} \binom{A}{G} \text{CA} \binom{A}{G} \text{TA},$$

were chemically synthesized according to the aforesaid solid-phase phosphotriester method.

5. Isolation and identification of bacterial clones containing the human t-PA cDNA sequence

A mixture of the eight types of l4-nucleotide oligomers (l4mers) synthesized in the above-described manner was treated with T4-polynucleotide kinase to label their 5'-end with $^{32}$P. On the other hand, the previously prepared filters containing a cDNA library were placed in a solution containing 6 x SSC (1 x SSC; 0.15M NaCl, 0.015M sodium succinate, pH 7.0), 10 x Denhardt's solution [1 x Denhardt's solution; 0.02% bovine serum albumin (fraction V), 0.02% polyvinyl pyrrolidone (3.6 x 10$^3$-4 x 10$^4$ dalton), 0.02% Ficoll (4 x 10$^5$ dalton)], 0.1% SDS and 100 $\mu$g/ml ultrasonically-treated salmon sperm DNA, and prehybridized at room temperature for 2 hours. Then, they were placed in a solution containing 6 x SSC, 10 x Dehnhardt's solution, and a labelled probe with a concentration of 5 x 10$^6$ counts/min/ml, and hybridized at room temperature overnight. Thereafter, the filters were washed three times at room temperature for 5 minutes in a solution containing 6 x SSC and 0.1% SDS, and then washed three times at 30°C for 5 minutes in the same solution.

After the washed filters were air-dried, they were used to expose Kodak XR-5 X-ray film for 16 hours with the aid of Dupont Kronex Lightening Plus intensifying screens.

From 14 colonies exhibiting a positive hybridization reaction, plasmid DNA was isolated according to a modification of the aforesaid method of Birnboim and Doly. Using this plasmid DNA, the base sequence of its cDNA insert was determined according to the aforesaid method of Maxam and Gilbert.

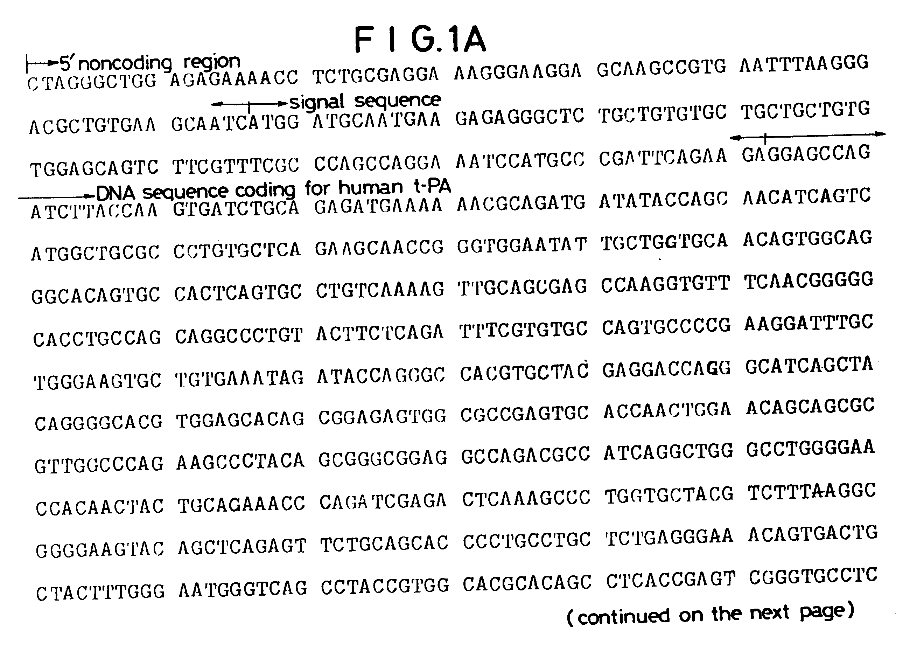

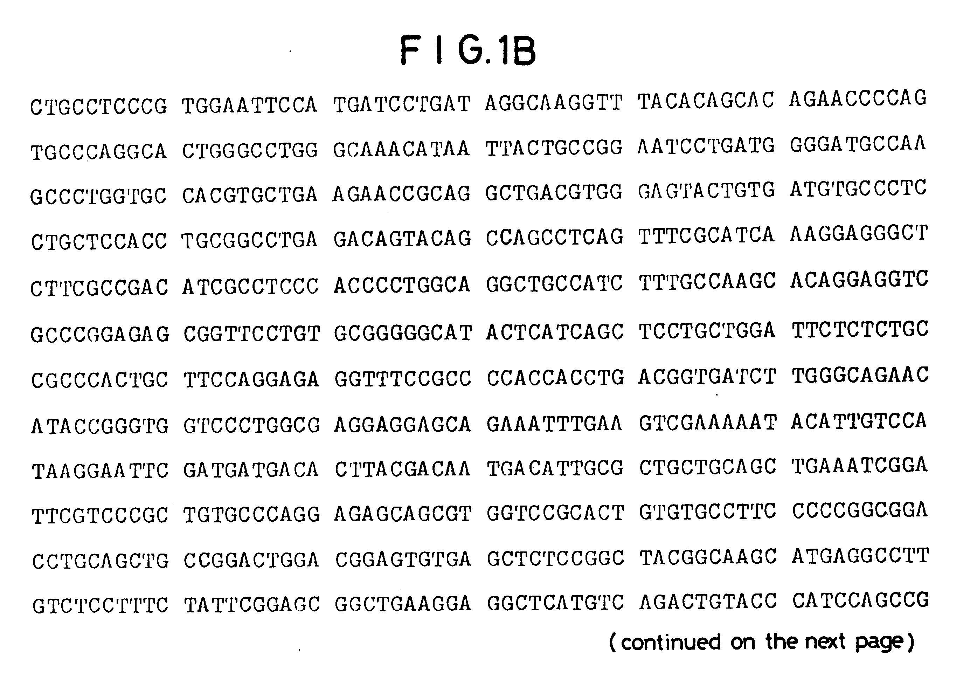

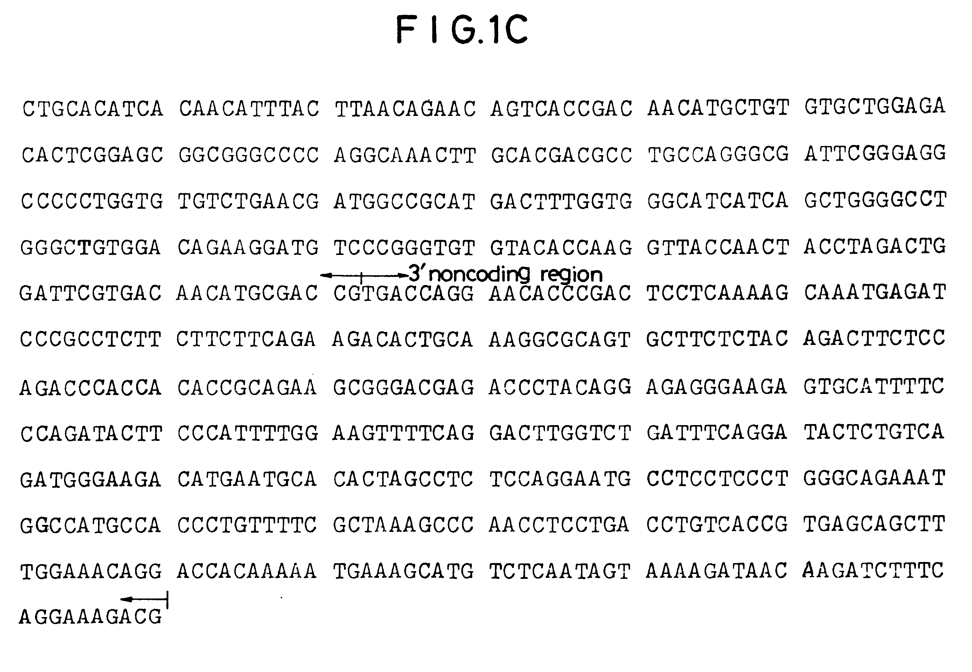

By comparing the amino acid sequence determined from the DNA base sequence of clone T-I (which was one of the 14 colonies) with the previously determined amino acid sequence of purified human t-PA, it was found that this clone contained the cDNA coding for human t-PA. Fig. 1 illustrates the base sequence of this cDNA insert. This insert had a size of 2170 bp and contained an open reading frame of 1686 bp, a 5' untrnaslated region of 76 bp and a 3' untranslated region of 408 bp. When this DNA sequence was compared with the DNA sequence coding for human t-PA which had been isolated from Bowes melanoma, they were different from each other at 3 points in the 5' untranslated region, at 1 Point in the region coding for human t-PA, and at 5 points in the 3' untranslated region.

Example 2 [Expression of the t-PA gene in mammalian cells by using the mouse metallothionein (MMT) promoter]

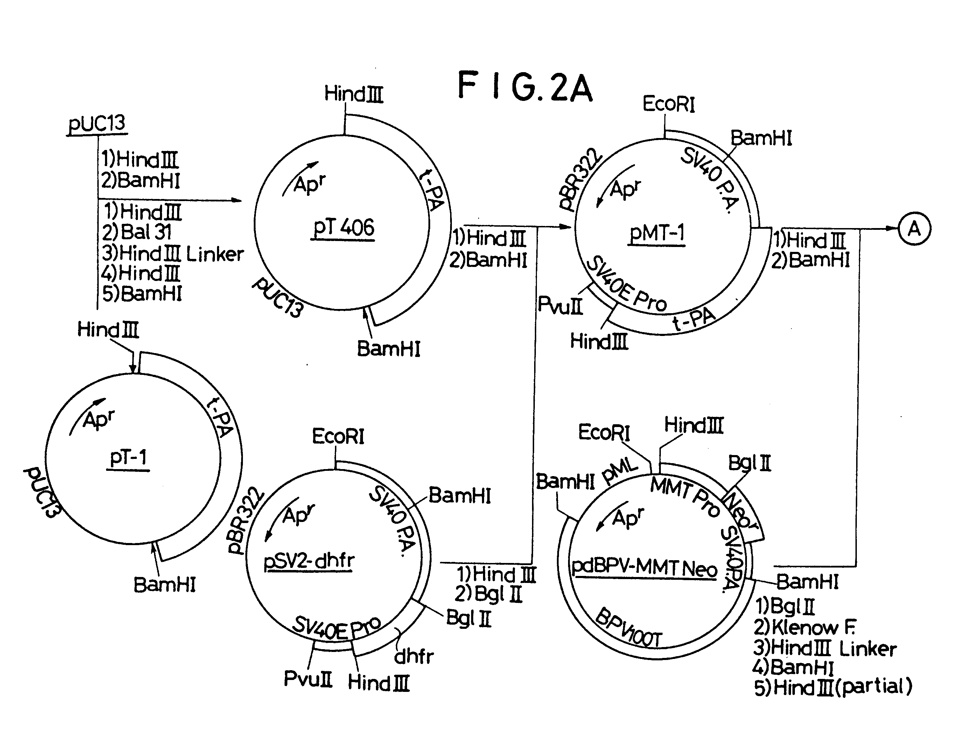

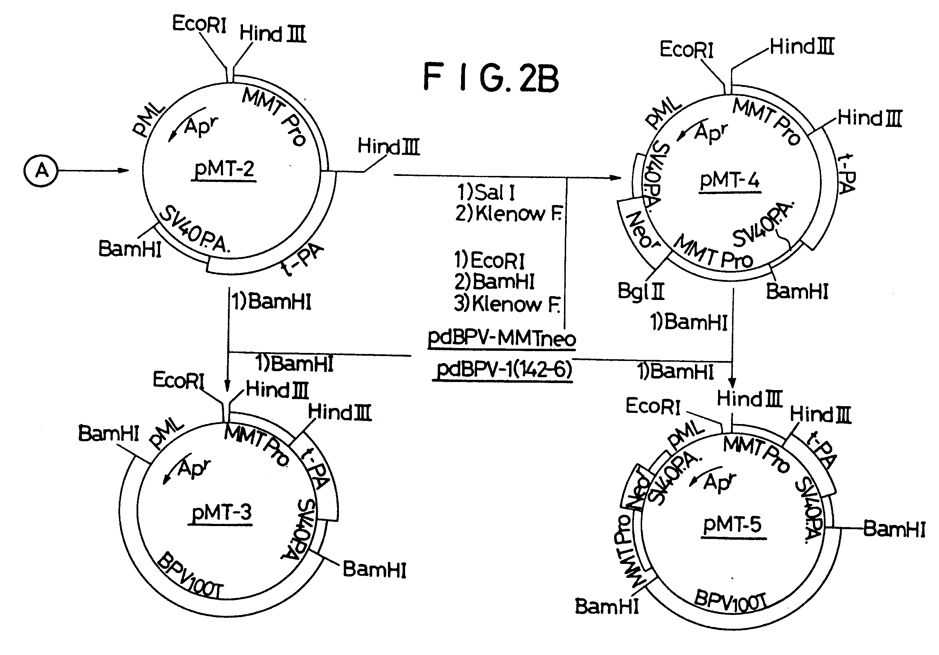

According to the procedure illustrated in Fig. 2, a number of plasmids to be introduced into mammalian cells were prepared in the following manner.

1. Preparation of the plasmid pMT-1

First, the plasmid pT-1 was isolated from the clone T-I obtained in Example 1. In order to remove the double strand of GC base pairs formed during cDNA synthesis, 25 $\mu$g of the PT-1 was cleaved with HindIII and then treated with 0.4U of Bal31 at 37°C for 2 hours. Using T4 DNA ligase, a HindIII linker was joined to the resulting DNA fragment, followed by cleavage with HindIII and BamHI. From the resulting DNA fragment mixture, a DNA fragment containing the t-PA gene was separated and recovered by polyacrylamide gel electrophoresis.

On the other hand, pUC13 was cleaved with HindIII and BamHI. From the resulting DNA fragment mixture, a DNA fragment of about 2700 bp was separated and recovered by low-melting agarose gel electrophoresis.

Using T4 DNA ligase, the above DNA fragment (of about 2700 bp) and the previously prepared DNA fragment containing the t-PA gene were joined to each other. The recombinant DNA so formed was used to transform E. coli HB101 in the same manner as described in Example 1.

Plasmids were prepared from ten of the resulting ampicillin-resistant colonies and used to determine the DNA base sequence of the 5' untranslated region of the t-PA gene.

The plasmid pT406 prepared from clone T-406, which was one of the aforesaid ten colonies, contained a 5' untranslated region of 39 bp. 20 $\mu$g of pT406 was cleaved with HindIII and BamHI. From the resulting DNA fragment mixture, a DNA fragment of about 2200 bp containing the t-PA gene was separated and

EP 0 225 177 B1

recovered by polyacrylamide gel electrophoresis.

On the other hand, 10 μg of the plasmid pSV2-dhfr (ATCC 37146) was cleaved with HindIII and BamHI. From the resulting DNA fragment mixture, a DNA fragment of about 4200 bp freed of the dhfr gene was separated and recovered by low-melting agarose gel electrophoresis.

Using T4 DNA ligase, 0.2 μg of the above DNA fragment (of about 4200 bp) and 0.1 μg of the previously isolated fragment of about 2200 bp containing the human t-PA gene were joined to each other. The recombinant DNA so formed was used to transform E. coli HB101 in the same manner as described above and thereby obtain ampicillin-resistant colonies. Small amounts of plasmids were isolated from these colonies and treated with restriction enzymes. On the basis of their cleavage patterns, the clones having the desired plasmid pMT-1 were identified and the plasmid pMT-1 was isolated therefrom.

2. Preparation of the plasmid pMT-2

10 μg of the plasmid pMT-1 prepared in the foregoing Section 1 was cleaved with HindIII and BamHI. From the resulting DNA fragment mixture, a DNA fragment of about 3 kbp containing the t-PA gene and the poly(A) signal of SV40 was separated and recovered by low-melting agarose gel electrophoresis.

On the other hand, a DNA fragment containing pML [M. Lusky and M. Botchan, Nature, 293, 79 (1981)] and the MMT promoter was prepared from the plasmid pdBPV-MMTneo (342-12) [M. Law et al., Molecular and Cellular Biology, 3, 2110 (1983)]. Specifically, 10 μg of pdBPV-MMTneo (342-12) was cleaved with BglII. After this treatment, 5 units of the Klenow fragment of DNA polymerase I and 0.1mM each of dATP, dCTP, dGTP and TTP were added to the resulting DNA fragment mixture. This reaction mixture was incubated at 37°C for 10 minutes to convert the cohesive ends produced by cleavage with BglII to blunt ends. After completion of the reaction, the reaction product was purified by extraction with phenol, treatment with chloroform, and precipitation with ethanol. Using T4 DNA ligase, 0.05 μg of a HindIII linker was joined to the purified product, followed by cleavage with BamHI and then partial cleavage with HindIII. From the resulting DNA fragment mixture, a fragment containing pML and the MMT promoter was separated and recovered by low-melting agarose gel electrophoresis.

Using T4 DNA ligase, 0.05 μg of the above DNA fragment and 0.04 μg of the previously prepared DNA fragment of 3 kbp containing the human t-PA gene and the poly(A) signal of SV40 were joined to each other. The recombinant DNA so formed was used to transform E. coli HB101 in the same manner as described above and thereby obtain ampicillin-resistant colonies.

Small amounts of plasmids were isolated from these colonies and treated with restriction enzymes. On the basis of their cleavage patterns, the clone having the desired plasmid pMT-2 was identified and the plasmid pMT-2 was isolated therefrom.

3. Preparation of the plasmid pMT-3

The plasmid pMT-2 prepared in the preceding Section 2 was cleaved with BamHI. The resulting DNA fragment was treated with calf intestinal alkaline phosphatase (hereinafter referred to as CIP) to effect dephosphorylation of the 5'-terminus.

On the other hand, pdBPV-1 (142-6) (ATCC 37134) was cleaved with BamHI. From the resulting DNA fragment mixture, a BPV$_{100T}$ DNA fragment of about 8 kbp was separated and recovered by low-melting agarose gel electrophoresis.

Using T4 DNA ligase, 0.05 μg of the BPV$_{100T}$ DNA fragment and 0.04 μg of the previously BamHI-cleaved and CIP-treated pMT-2 were joined to each other. The recombinant DNA so formed was used to transform E. coli HB101 in the same manner as described above and thereby obtain ampicillin-resistant colonies. Small amounts of plasmids were isolated from these colonies and treated with restriction enzymes. On the basis of their cleavage patterns, the clone having the desired plasmid pMT-3 was identified and the plasmid pMT-3 was isolated therefrom.

4. Preparation of the plasmid pMT-4

The plasmid pMT-2 prepared in the preceding Section 2 was partially cleaved with Sal I, treated with CIP, and then treated with the Klenow fragment to convert its cohesive ends into blunt ends. This DNA fragment was used as a vector.

On the other hand, pdBPV-MMTneo (342-12) was cleaved with EcoRI and BamHI, and then treated with the Klenow fragment of DNA polymerase I to convert the cohesive ends produced by the cleavage into blunt ends. From the resulting DNA fragment mixture, a DNA fragment of about 4 kbp containing the neo$^r$

gene was separated and recovered by low-melting agarose gel electrophoresis.

Using T4 DNA ligase, 0.05 $\mu$g of the above DNA fragment (of about 4 kbp) and 0.05 $\mu$g of the previously prepared vector were joined to each other. The recombinant DNA so formed was used to transform E. coli HB101 in the same manner as described above. Thereafter, the same procedure as described in the preceding Section 3 was repeated to identify the clone having the desired plasmid pMT-4 and isolate the plasmid pMT-4 therefrom.

### 5. Preparation of the plasmid pMT-5

The plasmid pMT-4 prepared in the preceding Section 4 was cleaved with BamHI and then treated with CIP. Using T4 DNA ligase, 0.05 $\mu$g of the resulting DNA fragment and 0.05 $\mu$g of the BPV$_{100T}$ DNA fragment prepared in the preceding Section 3 were joined to each other. The recombinant DNA so formed was used to transform E. coli HB101 in the same manner as described above. Thereafter, the same procedure as described in the preceding Section 4 was repeated to identify the clone having the desired plasmid pMT-5 and isolate the plasmid pMT-5 therefrom.

### 6. Introduction of the MMT promoter-bearing plasmids into mammalian cells and expression of the t-PA gene

The MMT promoter-bearing plasmids pMT-2 to -5 for the expression of the t-PA gene, prepared in the preceding Sections 2-5, were separately introduced into mammalian cells. The introduction was carried out according to a modification of the calcium phosphate precipitation method developed by Graham and Van der Eb. Specifically, a solution containing 2 x HBS [50mM Hepes (pH 7.1), 280mM NaCl], 1.4mM NaH$_2$PO$_4$ and 1.4mM Na$_2$HPO$_4$ was prepared. While sterile air was being sucked through the solution, an equal volume of a solution containing one of the plasmids for the expression of the t-PA gene and 250mM CaCl$_2$ was added dropwise thereto so as to form a plasmid-CaPO$_4$ precipitate. After completion of the addition, the solution was allowed to stand for 20 minutes. Then, the precipitate was suspended with a pipet and the resulting suspension was added dropwise to host cells cultured in a flask. After the host cells were grown at 37°C for several hours in a CO$_2$ incubator, the medium was removed and a solution containing 15% glycerol and 2 x HBS was added, followed by standing at 37°C for 3 minutes. After this solution was removed, a fresh medium was added and the host cells were grown for 2 days in a CO$_2$ incubator. Then, the host cells were transferred to a plate containing a fresh medium and grown for a week. In this case, a medium containing the antibiotic substance G418 was used because the neo resistance gene was utilized as a marker for the selection of transformants.

Thereafter, the medium was removed from the plate. According to the Jones et al. [P. Jones et al., Cell, 5, 323 (1975)],a casein agar layer (instead of fibrin) was placed over the host cells and the plate was incubated overnight. The t-PA producing clones which exhibited a transparent dissolution zone in the casein agar layer were selected. Then, clones highly productive of t-PA were obtained by culturing each clone and assaying the human t-PA activity of the supernatant of the resulting culture. The results thus obtained are shown in Table 2. These highly productive clones were also cultured in a medium containing 1 $\mu$M Cd$^{++}$ or 50 $\mu$M Zn$^{++}$ and the human t-PA activity of the supernatant of the resulting culture was assayed. The results thus obtained are also shown in Table 2.

As a result, clone MT-325 exhibited the highest human t-PA activity. Specifically, this clone produced 110 units/ml/day of human t-PA in the absence of Cd$^{++}$ or Zn$^{++}$ and 305 or 310 units/ml/day of human t-PA in the presence of Cd$^{++}$ or Zn$^{++}$, respectively.

## Table 2

### Clones Expressing Human t-PA by Use of the MMT Promoter

| Clone No. | Expression plasmid | Amount of expression of human t-PA (units/ml/day) | | |
|---|---|---|---|---|
| | | No addition | Addition of 1µM Cd++ | Addition of 50µM Zn++ |
| MT-103 | pMT-2+pSV2-neo (ATCC 37149) | 20 | 60 | 65 |
| MT-157 | " | 25 | 80 | 70 |
| MT-207 | pMT-4 | 35 | 110 | 120 |
| MT-302 | pMT-3+pSV2-neo | 90 | 265 | 275 |
| MT-325 | " | 110 | 310 | 305 |
| MT-387 | " | 85 | 255 | 270 |
| MT-421 | pMT-5 | 60 | 165 | 170 |
| MT-446 | " | 100 | 300 | 300 |
| MT-474 | " | 75 | 260 | 250 |

(Note) In all cases, the mouse C127I cell was used as the host, and transformants were selected with reference to G418 resistance.

Example 3 [Expression of the human t-PA gene in mammalian cells by using the human metallothionein (hMT) promoter]

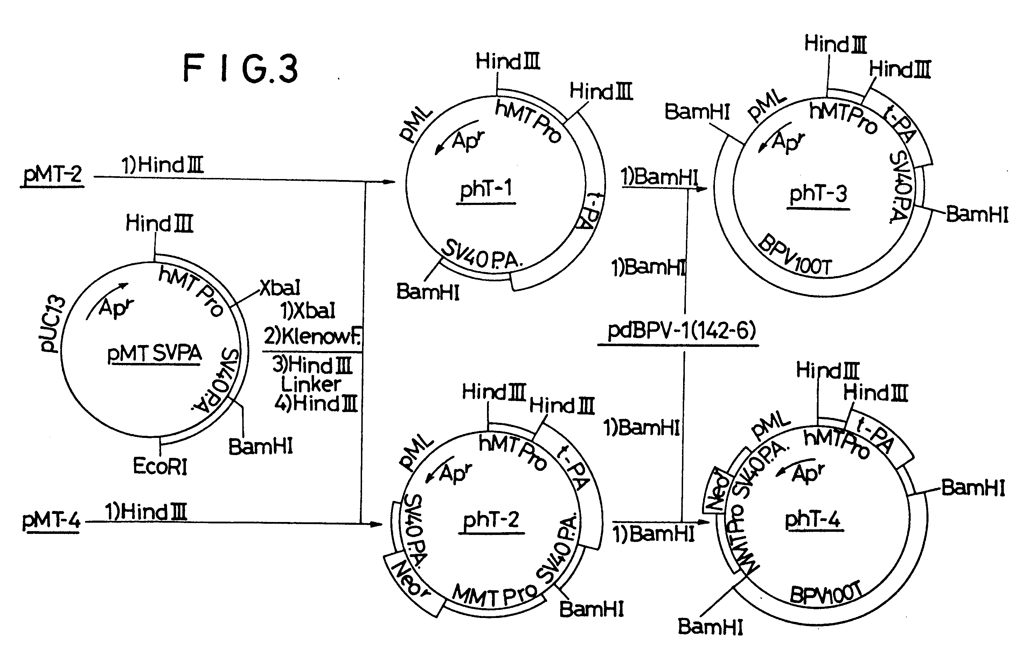

According to the procedure illustrated in Fig. 3, a number of plasmids having the hMT promoter were prepared in the following manner.

14

1. Preparation of the plasmid phT-1

First, the plasmid pMTSVPA (obtained from Dr. M. Karin, School of Medicine, University of Southern California, U.S.A.) was cleaved with XbaI and then treated with the Klenow fragment of DNA polymerase I to convert the cohesive ends produced by the cleavage into blunt ends.

Using T4 DNA ligase, a HindIII linker was joined to the resulting DNA fragment, followed by cleavage with HindIII. From the resulting DNA fragment mixture, a DNA fragment of about 840 bp corresponding to the hMT promoter was separated and recovered by low-melting agarose gel electrophoresis.

On the other hand, the plasmid pMT-2 obtained in Section 2 of Example 2 was cleaved with HindIII and then treated with CIP. From the resulting DNA fragment mixture, a DNA fragment of about 5.5 kbp comprising the plasmid pMT-2 devoid of the MMT promoter was separated and recovered by low-melting agarose gel electrophoresis.

Using T4 DNA ligase, the above DNA fragment (of about 5.5 kbp) and the previously prepared DNA fragment having the hMT promoter were joined to each other. The recombinant DNA so formed was used to transform E. coli HB101 in the same manner as described above.

Small amounts of plasmids were isolated from the resulting ampicillin-resistant colonies and treated with restriction enzymes. On the basis of their cleavage patterns, the clone having the desired plasmid phT-1 was identified and the plasmid phT-1 was isolated therefrom.

2. Preparation of the plasmid phT-2

The plasmid phT-2 was isolated in the same manner as described in the preceding Section 1, except that the plasmid pMT-4 obtained in Section 4 of Example 1 was used in place of the plasmid pMT-2.

3. Preparation of the plasmid phT-3

The plasmid phT-1 prepared in the preceding Section 1 was cleaved with BamHI and then treated with CIP. Using T4 DNA ligase, the resulting DNA fragment and the $BPV_{100T}$ DNA fragment of about 8 kbp obtained in the preparation of the plasmid pMT-3 were joined to each other. The recombinant DNA so formed was used to transform E. coli HB101 in the same manner as described above. Thereafter, the same procedure as described in the preceding Section 2 was repeated to isolate the plasmid phT-3.

4. Preparation of the plasmid phT-4

The plasmid phT-4 was isolated in the same manner as described in the preceding Section 3, except that the plasmid phT-2 obtained in the preceding Section 2 was used in place of the plasmid phT-1.

5. Introduction of the hMT promoter-bearing plasmids into mammalian cells and expression of the human t-PA gene

The hMT promoter-bearing plasmids phT-1 to -4 for the expression of the t-PA gene, prepared in the preceding Sections 1-4, were separately introduced into mammalian cells. The introduction and the screening of clones highly productive of t-PA were carried out in the same manner as described in Section 6 of Example 2. The results thus obtained are shown in Table 3. The highly productive clones were also cultured in a medium containing $1\mu M$ $Cd^{++}$, $50\mu M$ $Zn^{++}$ or $1\mu M$ dexamethasone, and the human t-PA activity of the supernatant of the resulting culture was assayed. The results thus obtained are also shown in Table 3.

As a result, clone hT-382 exhibited the highest human t-PA activity. Specifically, this clone produced 105 units/ml/day of human t-PA in the absence of $Cd^{++}$, $Zn^{++}$ or dexamethasone and 330-340 units/ml/day of human t-PA in the presence of $Cd^{++}$, $Zn^{++}$ or dexamethasone.

## Table 3

### Clones Expressing Human t-PA by Use of the hMT Promoter

| Clone No. | Expression plasmid | Amount of expression of human t-PA (units/ml/day) | | | |
|---|---|---|---|---|---|
| | | No addition | Addition of 1µM Cd$^{++}$ | Addition of 50µM Zn$^{++}$ | Addition of 1µM dexa-methasone |
| hT-131 | phT-1+pSV2-neo | 15 | 55 | 50 | 60 |
| hT-147 | " | 35 | 125 | 125 | 130 |
| hT-213 | phT-2 | 40 | 115 | 130 | 100 |
| hT-236 | " | 25 | 85 | 90 | 90 |
| hT-320 | phT-3+pSV2-neo | 60 | 230 | 215 | 240 |
| hT-341 | " | 70 | 250 | 245 | 260 |
| hT-382 | " | 105 | 330 | 340 | 335 |
| hT-387 | " | 85 | 250 | 260 | 255 |
| hT-418 | phT-4 | 90 | 295 | 300 | 320 |
| hT-455 | " | 90 | 300 | 280 | 315 |

(Note) In all cases, the mouse C127I cell was used as the host, and transformants were selected with reference to G418 resistance.

## Claims

1. Recombinant DNA vector comprising;
    (a) a portion capable of self-replication in mammalian cells,

16

(b) a promoter selected from mouse methallothionein promoter and human metallothionein promoter, and

(c) the DNA fragment containing the sequence coding for human tissue plasminogen activator (t-PA) derived from a human fetal epithelium cell, said t-PA comprising 530 amino acids, the N-terminal of which begins with glycine, wherein one strand of said DNA sequence has the following base sequence:

```
         GGAG        CCAGATCTTA    CCAAGTGATC    TGCAGAGATG

      AAAAAACGCA     GATGATATAC    CAGCAACATC    AGTCATGGCT

      GCGCCCTGTG     CTCAGAAGCA    ACCGGGTGGA    ATATTGCTGG

      TGCAACAGTG     GCAGGGCACA    GTGCCACTCA    GTGCCTGTCA

      AAAGTTGCAG     CGAGCCAAGG    TGTTTCAACG    GGGGCACCTG

      CCAGCAGGCC     CTGTACTTCT    CAGATTTCGT    GTGCCAGTGC

      CCCGAAGGAT     TTGCTGGGAA    GTGCTGTGAA    ATAGATACCA

      GGGCCACGTG     CTACGAGGAC    CAGGGCATCA    GCTACAGGGG

      CACGTGGAGC     ACAGCGGAGA    GTGGCGCCGA    GTGCACCAAC

      TGGAACAGCA     GCGCGTTGGC    CCAGAAGCCC    TACAGCGGGC

      GGAGGCCAGA     CGCCATCAGG    CTGGGCCTGG    GGAACCACAA

      CTACTGCAGA     AACCCAGATC    GAGACTCAAA    GCCCTGGTGC

      TACGTCTTTA     AGGCGGGGAA    GTACAGCTCA    GAGTTCTGCA

      GCACCCCTGC     CTGCTCTGAG    GGAAACAGTG    ACTGCTACTT
```

```
TGGGAATGGG    TCAGCCTACC    GTGGCACGCA    CAGCCTCACC

GAGTCGGGTG    CCTCCTGCCT    CCCGTGGAAT    TCCATGATCC

TGATAGGCAA    GGTTTACACA    GCACAGAACC    CCAGTGCCCA

GGCACTGGGC    CTGGGCAAAC    ATAATTACTG    CCGGAATCCT

GATGGGGATG    CCAAGCCCTG    GTGCCACGTG    CTGAAGAACC

GCAGGCTGAC    GTGGGAGTAC    TGTGATGTGC    CCTCCTGCTC

CACCTGCGGC    CTGAGACAGT    ACAGCCAGCC    TCAGTTTCGC

ATCAAAGGAG    GGCTCTTCGC    CGACATCGCC    TCCCACCCCT

GGCAGGCTGC    CATCTTTGCC    AAGCACAGGA    GGTCGCCCGG

AGAGCGGTTC    CTGTGCGGGG    GCATACTCAT    CAGCTCCTGC

TGGATTCTCT    CTGCCGCCCA    CTGCTTCCAG    GAGAGGTTTC

CGCCCCACCA    CCTGACGGTG    ATCTTGGGCA    GAACATACCG

GGTGGTCCCT    GGCGAGGAGG    AGCAGAAATT    TGAAGTCGAA

AAATACATTG    TCCATAAGGA    ATTCGATGAT    GACACTTACG

ACAATGACAT    TGCGCTGCTG    CAGCTGAAAT    CGGATTCGTC

CCGCTGTGCC    CAGGAGAGCA    GCGTGGTCCG    CACTGTGTGC

CTTCCCCCGG    CGGACCTGCA    GCTGCCGGAC    TGGACGGAGT

GTGAGCTCTC    CGGCTACGGC    AAGCATGAGG    CCTTGTCTCC

TTTCTATTCG    GAGCGGCTGA    AGGAGGCTCA    TGTCAGACTG

TACCCATCCA    GCCGCTGCAC    ATCACAACAT    TTACTTAACA

GAACAGTCAC    CGACAACATG    CTGTGTGCTG    GAGACACTCG

GAGCGGCGGG    CCCCAGGCAA    ACTTGCACGA    CGCCTGCCAG

GGCGATTCGG    GAGGCCCCCT    GGTGTGTCTG    AACGATGGCC

GCATGACTTT    GGTGGGCATC    ATCAGCTGGG    GCCTGGGCTG

TGGACAGAAG    GATGTCCCGG    GTGTGTACAC    CAAGGTTACC

AACTACCTAG    ACTGGATTCG    TGACAACATG    CGACCG,
```

said DNA fragment being operably linked to the promoter.

**2.** A microbial or mammalian cell transformed with the recombinant DNA of claim 1.

**3.** A process for the production of human tissue plasminogen activator comprising the step of culturing the mammalian cell of claim 2 in the presence of a metal ion as an inducer for the mouse metallothionein promoter or the human metallothionein promoter.

**Patentansprüche**

**1.** Recombinanter DNA-Vektor, umfassend:
a) einen Abschnitt, der zur Selbst-Replikation in Säugetierzellen fähig ist,
b) einen Promotor, der aus Mäuse-Metallothioneinpromoter und menschlichem Metallothioneinpromotor ausgewählt ist, und
c) das DNA-Fragment, das die Sequenz enthält, die für menschlichen Gewebe-Plasminogen-Aktivator (t-PA) codiert, der von einer menschlichen fötalen Epithelzelle abgeleitet ist, wobei dieser t-PA 530 Aminosäuren umfaßt, deren N-Terminus mit Glycin beginnt, worin ein Strang dieser DNA-Sequenz die folgende Basen-Sequenz aufweist:

| | | | |
|---|---|---|---|
| GGAG | CCAGATCTTA | CCAAGTGATC | TGCAGAGATG |
| AAAAAACGCA | GATGATATAC | CAGCAACATC | AGTCATGGCT |
| GCGCCCTGTG | CTCAGAAGCA | ACCGGGTGGA | ATATTGCTGG |
| TGCAACAGTG | GCAGGGCACA | GTGCCACTCA | GTGCCTGTCA |
| AAAGTTGCAG | CGAGCCAAGG | TGTTTCAACG | GGGGCACCTG |
| CCAGCAGGCC | CTGTACTTCT | CAGATTTCGT | GTGCCAGTGC |
| CCCGAAGGAT | TTGCTGGGAA | GTGCTGTGAA | ATAGATACCA |
| GGGCCACGTG | CTACGAGGAC | CAGGGCATCA | GCTACAGGGG |
| CACGTGGAGC | ACAGCGGAGA | GTGGCGCCGA | GTGCACCAAC |
| TGGAACAGCA | GCGCGTTGGC | CCAGAAGCCC | TACAGCGGGC |
| GGAGGCCAGA | CGCCATCAGG | CTGGGCCTGG | GGAACCACAA |
| CTACTGCAGA | AACCCAGATC | GAGACTCAAA | GCCCTGGTGC |
| TACGTCTTTA | AGGCGGGGAA | GTACAGCTCA | GAGTTCTGCA |
| GCACCCCTGC | CTGCTCTGAG | GGAAACAGTG | ACTGCTACTT |

TGGGAATGGG     TCAGCCTACC     GTGGCACGCA     CAGCCTCACC

GAGTCGGGTG     CCTCCTGCCT     CCCGTGGAAT     TCCATGATCC

TGATAGGCAA     GGTTTACACA     GCACAGAACC     CCAGTGCCCA

GGCACTGGGC     CTGGGCAAAC     ATAATTACTG     CCGGAATCCT

GATGGGGATG     CCAAGCCCTG     GTGCCACGTG     CTGAAGAACC

GCAGGCTGAC     GTGGGAGTAC     TGTGATGTGC     CCTCCTGCTC

CACCTGCGGC     CTGAGACAGT     ACAGCCAGCC     TCAGTTTCGC

ATCAAAGGAG     GGCTCTTCGC     CGACATCGCC     TCCCACCCCT

GGCAGGCTGC     CATCTTTGCC     AAGCACAGGA     GGTCGCCCGG

AGAGCGGTTC     CTGTGCGGGG     GCATACTCAT     CAGCTCCTGC

TGGATTCTCT     CTGCCGCCCA     CTGCTTCCAG     GAGAGGTTTC

CGCCCCACCA     CCTGACGGTG     ATCTTGGGCA     GAACATACCG

GGTGGTCCCT     GGCGAGGAGG     AGCAGAAATT     TGAAGTCGAA

AAATACATTG     TCCATAAGGA     ATTCGATGAT     GACACTTACG

ACAATGACAT     TGCGCTGCTG     CAGCTGAAAT     CGGATTCGTC

CCGCTGTGCC     CAGGAGAGCA     GCGTGGTCCG     CACTGTGTGC

CTTCCCCCGG     CGGACCTGCA     GCTGCCGGAC     TGGACGGAGT

GTGAGCTCTC     CGGCTACGGC     AAGCATGAGG     CCTTGTCTCC

TTTCTATTCG     GAGCGGCTGA     AGGAGGCTCA     TGTCAGACTG

TACCCATCCA     GCCGCTGCAC     ATCACAACAT     TTACTTAACA

GAACAGTCAC     CGACAACATG     CTGTGTGCTG     GAGACACTCG

GAGCGGCGGG     CCCCAGGCAA     ACTTGCACGA     CGCCTGCCAG

GGCGATTCGG     GAGGCCCCT     GGTGTGTCTG     AACGATGGCC

GCATGACTTT     GGTGGGCATC     ATCAGCTGGG     GCCTGGGCTG

TGGACAGAAG     GATGTCCCGG     GTGTGTACAC     CAAGGTTACC

AACTACCTAG     ACTGGATTCG     TGACAACATG     CGACCG,

wobei dieses DNA-Fragment funktionsbereit an den Promotor gebunden ist.

**2.** Eine mikrobielle oder Säugetierzelle, die mit der rekombinanten DNA nach Anspruch 1 transformiert ist.

**3.** Ein Verfahren zur Herstellung von menschlichem Gewebe-Plasminogen-Aktivator, das den Verfahrensschritt des Züchtens der Säugetierzelle nach Anspruch 2 in Anwesenheit eines Metallions als eines Induktors für den Mäuse-Metallothioneinpromotor oder den meschlichen Metallothioneinpromotor umfaßt.

**Revendications**

**1.** Vecteur d'ADN recombinant, comprenant :
(a) une partie assurant l'auto-réplication dans des cellules de mammifère,
(b) un promoteur choisi parmi le promoteur d'un gène de la métallothionéine murine et le promoteur d'un gène de la métallothionéine humaine, et
(c) le fragment d'ADN contenant la séquence codant pour l'activateur du plasminogène tissulaire humain (t-PA) obtenu à partir d'une cellule de l'épithélium foetal humain, ledit t-PA comprenant 530 acides aminés, dont la partie amino-terminale commence par la glycine, fragment dans lequel un brin de ladite séquence d'ADN présente la séquence de base suivante :

```
GGAG        CCAGATCTTA    CCAAGTGATC    TGCAGAGATG

AAAAAACGCA  GATGATATAC    CAGCAACATC    AGTCATGGCT

GCGCCCTGTG  CTCAGAAGCA    ACCGGGTGGA    ATATTGCTGG

TGCAACAGTG  GCAGGGCACA    GTGCCACTCA    GTGCCTGTCA

AAAGTTGCAG  CGAGCCAAGG    TGTTTCAACG    GGGGCACCTG

CCAGCAGGCC  CTGTACTTCT    CAGATTTCGT    GTGCCAGTGC

CCCGAAGGAT  TTGCTGGGAA    GTGCTGTGAA    ATAGATACCA

GGGCCACGTG  CTACGAGGAC    CAGGGCATCA    GCTACAGGGG

CACGTGGAGC  ACAGCGGAGA    GTGGCGCCGA    GTGCACCAAC

TGGAACAGCA  GCGCGTTGGC    CCAGAAGCCC    TACAGCGGGC

GGAGGCCAGA  CGCCATCAGG    CTGGGCCTGG    GGAACCACAA

CTACTGCAGA  AACCCAGATC    GAGACTCAAA    GCCCTGGTGC

TACGTCTTTA  AGGCGGGGAA    GTACAGCTCA    GAGTTCTGCA

GCACCCCTGC  CTGCTCTGAG    GGAAACAGTG    ACTGCTACTT
```

TCGGAATCGG    TCAGCCTACC    GTGGCACGCA    CAGCCTCACC

GAGTCGGGTG    CCTCCTGCCT    CCCGTGGAAT    TCCATGATCC

TGATAGGCAA    GGTTTACACA    GCACAGAACC    CCAGTGCCCA

GGCACTGGGC    CTGGGCAAAC    ATAATTACTG    CCGGAATCCT

GATGGGGATG    CCAAGCCCTG    GTGCCACGTG    CTGAAGAACC

GCAGGCTGAC    GTGGGAGTAC    TGTGATGTGC    CCTCCTGCTC

CACCTGCGGC    CTGAGACAGT    ACAGCCAGCC    TCAGTTTCGC

ATCAAAGGAG    GGCTCTTCGC    CGACATCGCC    TCCCACCCCT

GGCAGGCTGC    CATCTTTGCC    AAGCACAGGA    GGTCGCCCGG

AGAGCGGTTC    CTGTGCGGGG    GCATACTCAT    CAGCTCCTGC

TGGATTCTCT    CTGCCGCCCA    CTGCTTCCAG    GAGAGGTTTC

CGCCCCACCA    CCTGACGGTG    ATCTTGGGCA    GAACATACCG

GGTGGTCCCT    GGCGAGGAGG    AGCAGAAATT    TGAAGTCGAA

AAATACATTG    TCCATAAGGA    ATTCGATGAT    GACACTTACG

ACAATGACAT    TGCGCTGCTG    CAGCTGAAAT    CGGATTCGTC

CCGCTGTGCC    CAGGAGAGCA    GCGTGGTCCG    CACTGTGTGC

CTTCCCCCGG    CGGACCTGCA    GCTGCCGGAC    TGGACGGAGT

GTGAGCTCTC    CGGCTACGGC    AAGCATGAGG    CCTTGTCTCC

TTTCTATTCG    GAGCGGCTGA    AGGAGGCTCA    TGTCAGACTG

TACCCATCCA    GCCGCTGCAC    ATCACAACAT    TTACTTAACA

GAACAGTCAC    CGACAACATG    CTGTGTGCTG    GAGACACTCG

GAGCGGCGGG    CCCCAGGCAA    ACTTGCACGA    CGCCTGCCAG

GGCGATTCGG    GAGGCCCCCT    GGTGTGTCTG    AACGATGGCC

GCATGACTTT    GGTGGGCATC    ATCAGCTGGG    GCCTGGGCTG

TGGACAGAAG    GATGTCCCGG    GTGTGTACAC    CAAGGTTACC

AACTACCTAG    ACTGGATTCG    TGACAACATG    CGACCG,

ledit fragment d'ADN étant ligaturé au promoteur de façon à être fonctionnel.

**2.** Cellule microbienne ou de mammifère, transformée à l'aide de l'ADN recombinant selon la revendication 1.

3. Procédé de préparation de l'activateur du plasminogène tissulaire humain comprenant l'étape consistant à cultiver les cellules de mammifère selon la revendication 2 en présence d'un ion métallique comme inducteur du promoteur d'un gène de la métallothionéine murine ou du promoteur d'un gène de la métallothionéine humaine.

# F I G.1A

├──►5′noncoding region
CTAGGGCTGG AGAGAAAACC TCTGCGAGGA AAGGGAAGGA GCAAGCCGTG AATTTAAGGG

├───────►signal sequence
ACGCTGTGAA GCAATCATGG ATGCAATGAA GAGAGGGCTC TGCTGTGTGC TGCTGCTGTG

TGGAGCAGTC TTCGTTTCGC CCAGCCAGGA AATCCATGCC CGATTCAGAA GAGGAGCCAG

──────►DNA sequence coding for human t-PA
ATCTTACCAA GTGATCTGCA GAGATGAAAA AACGCAGATG ATATACCAGC AACATCAGTC

ATGGCTGCGC CCTGTGCTCA GAAGCAACCG GGTGGAATAT TGCTGGTGCA ACAGTGGCAG

GGCACAGTGC CACTCAGTGC CTGTCAAAAG TTGCAGCGAG CCAAGGTGTT TCAACGGGGG

CACCTGCCAG CAGGCCCTGT ACTTCTCAGA TTTCGTGTGC CAGTGCCCCG AAGGATTTGC

TGGGAAGTGC TGTGAAATAG ATACCAGGGC CACGTGCTAC GAGGACCAGG GCATCAGCTA

CAGGGGCACG TGGAGCACAG CGGAGAGTGG CGCCGAGTGC ACCAACTGGA ACAGCAGCGC

GTTGGCCCAG AAGCCCTACA GCGGGCGGAG GCCAGACGCC ATCAGGCTGG GCCTGGGGAA

CCACAACTAC TGCAGAAACC CAGATCGAGA CTCAAAGCCC TGGTGCTACG TCTTTAAGGC

GGGGAAGTAC AGCTCAGAGT TCTGCAGCAC CCCTGCCTGC TCTGAGGGAA ACAGTGACTG

CTACTTTGGG AATGGGTCAG CCTACCGTGG CACGCACAGC CTCACCGAGT CGGGTGCCTC

(continued on the next page)

EP 0 225 177 B1

# F I G.1B

CTGCCTCCCG TGGAATTCCA TGATCCTGAT AGGCAAGGTT TACACAGCAC AGAACCCCAG

TGCCCAGGCA CTGGGCCTGG GCAAACATAA TTACTGCCGG AATCCTGATG GGGATGCCAA

GCCCTGGTGC CACGTGCTGA AGAACCGCAG GCTGACGTGG GAGTACTGTG ATGTGCCCTC

CTGCTCCACC TGCGGCCTGA GACAGTACAG CCAGCCTCAG TTTCGCATCA AAGGAGGGCT

CTTCGCCGAC ATCGCCTCCC ACCCCTGGCA GGCTGCCATC TTTGCCAAGC ACAGGAGGTC

GCCCGGAGAG CGGTTCCTGT GCGGGGGCAT ACTCATCAGC TCCTGCTGGA TTCTCTCTGC

CGCCCACTGC TTCCAGGAGA GGTTTCCGCC CCACCACCTG ACGGTGATCT TGGGCAGAAC

ATACCGGGTG GTCCCTGGCG AGGAGGAGCA GAAATTTGAA GTCGAAAAAT ACATTGTCCA

TAAGGAATTC GATGATGACA CTTACGACAA TGACATTGCG CTGCTGCAGC TGAAATCGGA

TTCGTCCCGC TGTGCCCAGG AGAGCAGCGT GGTCCGCACT GTGTGCCTTC CCCCGGCGGA

CCTGCAGCTG CCGGACTGGA CGGAGTGTGA GCTCTCCGGC TACGGCAAGC ATGAGGCCTT

GTCTCCTTTC TATTCGGAGC GGCTGAAGGA GGCTCATGTC AGACTGTACC CATCCAGCCG

EP 0 225 177 B1

# FIG.1C

CTGCACATCA CAACATTTAC TTAACAGAAC AGTCACCGAC AACATGCTGT GTGCTGGAGA

CACTCGGAGC GGCGGGCCCC AGGCAAACTT GCACGACGCC TGCCAGGGCG ATTCGGGAGG

CCCCCTGGTG TGTCTGAACG ATGGCCGCAT GACTTTGGTG GGCATCATCA GCTGGGGCCT

GGGCTGTGGA CAGAAGGATG TCCCGGGTGT GTACACCAAG GTTACCAACT ACCTAGACTG

3' noncoding region

GATTCGTGAC AACATGCGAC CGTGACCAGG AACACCCGAC TCCTCAAAAG CAAATGAGAT

CCCGCCTCTT CTTCTTCAGA AGACACTGCA AAGGCGCAGT GCTTCTCTAC AGACTTCTCC

AGACCCACCA CACCGCAGAA GCGGGACGAG ACCCTACAGG AGAGGGAAGA GTGCATTTTC

CCAGATACTT CCCATTTTGG AAGTTTTCAG GACTTGGTCT GATTTCAGGA TACTCTGTCA

GATGGGAAGA CATGAATGCA CACTAGCCTC TCCAGGAATG CCTCCTCCCT GGGCAGAAAT

GGCCATGCCA CCCTGTTTTC GCTAAAGCCC AACCTCCTGA CCTGTCACCG TGAGCAGCTT

TGGAAACAGG ACCACAAAAA TGAAAGCATG TCTCAATAGT AAAAGATAAC AAGATCTTTC

AGGAAAGACG

EP 0 225 177 B1

F I G. 2A

F I G. 2B

EP 0 225 177 B1

F I G. 3